(19) Europäisches Patentamt
European Patent Office
Office européen des brevets

(11) Numéro de publication: **0 197 864**
**B1**

(12) # FASCICULE DE BREVET EUROPÉEN

(45) Date de publication du fascicule du brevet: **14.12.88**

(51) Int. Cl.⁴: **C 07 D 251/34**

(21) Numéro de dépôt: **86420057.1**

(22) Date de dépôt: **20.02.86**

(54) **Procédé de préparation de polyisocyanates polyisocyanurates par cyclotrimérisation catalytique de polyisocyanates.**

(30) Priorité: **25.03.85 FR 8504608**

(43) Date de publication de la demande:
**15.10.86 Bulletin 86/42**

(45) Mention de la délivrance du brevet:
**14.12.88 Bulletin 88/50**

(84) Etats contractants désignés:
**AT BE CH DE GB IT LI LU NL SE**

(56) Documents cité:
**EP-A-0 057 653**
**FR-A-2 522 667**

(73) Titulaire: **RHONE- POULENC CHIMIE, 25, quai Paul Doumer, F-92408 Courbevoie Cédex (FR)**

(72) Inventeur: **Robin, Jean, 21, rue Duguesclin, F-69006 Lyon (FR)**

(74) Mandataire: **Vignally, Noel, RHONE- POULENC INTERSERVICES Service Brevets Chimie Centre de Recherches de Saint- Fons B.P. 62, F-69192 Saint- Fons Cedex (FR)**

**EP 0 197 864 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne un perfectionnement à un procédé de préparation de polyisocyanates polyisocyanurates, par cyclotrimérisation catalytique partielle de polyisocyanates, cette réaction étant ultérieurement et volontairement arrêtée lorsque la teneur en trimère atteint la valeur désirée. Plus précisément, la présente invention concerne essentiellement un perfectionnement au procédé de désactivation du catalyseur, lors de l'emploi d'un catalyseur constitué par un composé à groupement aminosilylé.

Dans la demande de brevet européen publiée sous le No. 57 653 on a décrit un procédé de préparation de composés à groupements isocyanuriques monomères ou polymères et notamment de polyisocyanates polyisocyanurates par cyclotrimérisation catalytique d'isocyanates aliphatiques ou cycloaliphatiques en présence de composés à groupement(s) aminosilylés comme catalyseurs. Cette demande décrit plus particulièrement un procédé d'obtention de polyisonates polyisocyanurates par cyclotrimérisation catalytique partielle de polyisocyanates, notamment de diisocyanates, aliphatiques ou cycloaliphatiques; dans ce cas la réaction de cyclotrimérisation est arrêtée lorsque la teneur en groupements isocyanurates atteint la valeur désirée par inactivation du catalyseur à groupe aminossilyle. Divers moyens d'inactivation du catalyseur ont été proposés. Ainsi, dans la demande de brevet européen précitée, cette inactivation du catalyseur résulte de sa destruction au moyen d'un composé acide (acide chlorhydrique, chlorure d'acide) que l'on ajoute au milieu réactionnel. Dans le brevet français No. 82-03 799, publié sous le No. 2 522 667 la destruction du catalyseur est réalisée par addition d'un composé organique comportant au moins un groupe hydroxyle tel que les énols, les alcools, les phénols, les oximes ou les composés à groupe(s) hydroxysilyle(s), ou d'un composé résultant de la réaction d'un tel composé hydroxylé avec un réactif à groupe(s) isocyanate(s) tel que les carbamates, lesquels peuvent être générés "in situ" par réaction d'un composé hydroxylé avec l'excès de diisocyanate suivant les conditions d'addition du composé hydroxylé. Après arrêt de la réaction le diisocyanate en excès est séparé par distillation du polyisocyanate polyisocyanurate.

On a constaté que les produits ainsi obtenus peuvent parfois générer au stockage des diisocyanates libres gênants lors de l'utilisation des polyisocyanates polyisocyanurates dans des compositions de revêtement et notamment dans les vernis. Le problème du blocage de la réaction de cyclotrimérisation a donc été réexaminé avec pour objectif de le rendre plus simple et plus efficace au regard de la stabilité au stockage des polyisocyanates polyisocyanurates résultant de la cyclotrimérisation catalytique partielle des polyisocyanates aliphatiques, cycloaliphatiques ou aromatiques.

Plus spécifiquement un premier objet de la présente invention réside dans un procédé de blocage de la réaction de cyclotrimérisation plus simple que les procédés antérieurs.

Un deuxième objet de la présente invention réside dans un procédé de blocage de la réaction de cyclotrimérization qui conduit à des polyisocyanates polyisocyanurates de stabilité au stockage améliorée. Cette stabilité au stockage se manifeste par une plus grande inaptitude des polyisocyanates polyisocyanurates obtenus selon le procédé de l'invention à générer un diisocyanate monomère pendant le stockage et même lors d'un chauffage de longue durée à une température supérieure ou égale à 60°C.

Plus spécifiquement la présente invention a pour objet un procédé de préparation de polyisocyanates polyisocyanurates par cyclotrimérisation catalytique de polyisocyanates, en utilisant à titre de catalyseurs des composés à groupements aminosilylés puis, lorsque la teneur désirée en groupement isocyanurate est atteinte, destruction du catalyseur par addition d'un composé désactivateur caractérisé en ce que l'on utilise l'eau comme désactivateur.

Il est surprenant de constater que l'eau peut être utilisée pour détruire le catalyseur sans dommage pour le polyisocyanate polyisocyanurate alors que la facilité de réaction de l'eau avec les isocyanates avait conduit l'homme de métier à l'écarter au bénéfice des composés hydroxylés moins actifs.

La quantité d'eau peut varier dans de larges limites; en général il n'est cependant pas nécessaire de recourir à plus de 1 mole d'eau par groupe SI-N présent dans le catalyseur. Plus spécifiquement la quantité d'eau est comprise entre 0,25 et 1 mole par groupe aminosilyle et de préférence entre 0,4 et 0,8 mole par groupe aminosilyle.

La température à laquelle est ajoutée l'eau peut être comprise entre 0 et 100°C. Elle pourrait être supérieure à 100°C, mais cela ne se traduirait par aucun avantage particulier et conduirait à opérer sous pression. De préférence la température d'addition de l'eau est comprise entre 15 et 60°C.

Les composés aminosilylés utilisés comme catalyseurs de cyclotrimérisation dans le procédé selon l'invention sont ceux décrits et cités dans la demande de brevet européen No. 0 057 653 c'est-à-dire les composés ayant pour formule (I):

$$R_{(4-n)} \text{———} Si \left[ NR'R'' \right]_n$$

dans laquelle les divers symboles représentent respectivment:

-R: un radical monovalent de nature hydrocarbonée, aliphatique, cycloaliphatique, saturé ou insaturé, aryle,

aralkyle ou alkylaryle, éventuellement substitué par des atomes d'halogène ou des groupements CN, deux radicaux R pouvant constituer ensemble un radical hydrocarboné divalent,

-R': un radical monovalent choisi parmi les radicaux R, $SiR_3$, ou les radicaux amide de formule:

```
-CO-N-R'''
    |
    R
```

R''' représentant R ou $SiR_3$, R ayant la signification préalablement donnée, le radical R' pouvant éventuellement lorsqu'il ne représente pas un groupement amide ou un groupement $SiR_3$ constituer avec le radical R'' un radical hydrocarboné divalent,

-R'': un radical monovalent ayant la même signification que le radical R; ou un atome d'dydrogène lorsque R' n'est pas un radical amide,

-n: un nombre entier égal à 1 ou 2. Lorsque n est égal à 2, R' est un radical R.

Le catalyseur qui peut être un aminosilane, un diaminosilane, une silylurée ou un silazane est plus précisemment représenté par la formule (I), dans laquelle les divers symboles représentent respectivement:

-R: un radical alkyle, alkényle ou halogénoalkyle ou halogénoalkényle ayant de 1 à 5 atomes de carbone et comportant de 1 à 6 atomes de chlore et/ou de fluor, des radicaux cycloalkyles, cycloalkényles et halogénocycloalkyles, halogénocycloalkényles ayant de 3 à 8 atomes de carbone et contenant de 1 à 4 atomes de chlore et/ou de flour;
des radicaux aryles, alkylaryles et halogénoaryles ayant de 6 à 8 atomes de carbone et contenant de 1 à 4 atomes de chlore et/ou de flour;
des radicaux cyanoalkyles ayant de 3 à 4 atomes de carbone;

- deux symboles R portés par un même atome de silicium constituent entre eux un radical divalent ayant de 1 à 4 atomes de carbone.

-R': un radical monovalent choisi parmi les radicaux R, $SiR_3$, et CO(NR)-R''', R''' représentant R ou $SiR_3$, R ayant la signification plus précise qui vient juste d'être donnée ci-dessus;
R' pouvant constituer avec R'' un radical alkylène ayant de 4 à 6 atomes de carbone.

-R'': un radical alkyle ou alkényle ayant de 1 à 4 atomes de carbone, un radical cycloalkyole ou cycloalkényle ayant de 4 à 6 atomes de carbone nucléaire, un radical phényle ou tolyle ou xylyle, ou un atome d'hydrogène lorsque R' n'est pas un groupement amide.

Les composés aminosilylés, de formule (I), utilisés préférentiellement à titre de catalyseurs de cyclotrimérisation sont ceux dans la formule desquels les divers symboles représentent respectivement:

-R:

un radical méthyle, éthyle, propyle, vinyle, phényle, ces radicaux pouvant éventuellement être chlorés et/ou fluorés,

-R':

un radical alkyle choisi parmi les radicaux méthyle, éthyle, propyle ou butyle,
un radical $SiR_3$, R ayant l'une des significations qui vient juste d'être définies,
un radical carbonamide choisi parmi:
CO-NR-R
-CO-NR-$SiR_3$
R ayant l'une des significations qui vient juste d'être donnée

-R":

un radical méthyle, éthyle, propyle ou butyle, ou un atome d'hydrogène.

Enfin R' et R" peuvent ensemble constituer un radical butylène ou pentylène.

Comme on l'a déjà dit le catalyseur de cyclotrimérisation peut être un aminosilane, un diaminosilane, une monosilylurée, une disilylurée ou un silazane. Il est facile de déterminer la nature chimiqe exacte des divers composés à groupements aminosilylés utilisables, étant donné les diverses significations données précédément aux divers radicaux R, R', R", R'''. On notera en particulier que l'utilisation de silylurée obtenue par réaction d'amine secondaire et d'isocyanates-N silylé n'est pas envisagée. Ces silylurées sont impropres dans le procédé de cyclotrimérisation catalytique puisque libérant l'isocyanate silylé lors du chauffage.

Le composé à groupement aminosilylé sera un aminosilane lorsque n sera égal à 1 et lorsque R' représentera un radical R, les radicaux R et R" ayant l'une des significations préalablement données, deux radicaux R pouvant constituer ensemble un radical divalent ou bien encore R' et R" pouvant constituer ensemble un radical divalent. Parmi les aminosilanes on citera:

. le méthylamino triméthylsilane,
. le diméthylamino triméthylsilane,
. le diéthylamino triméthylsilane,
. le dibutylamino triméthylsilane,
. le diéthylamino diméthylvinylsilane,
. le diéthylamino dimétnylphénylsilane.

Le composé à groupement aminosilylé sera un diaminosilane lorsque n sera égal à 2 et lorsque R' représentera le radical R, les radicaux R et R" ayant l'une des significations préalablement données, deux radicaux R pouvant constituer ensemble un radical divalent ou bien encore R' et R" pouvant constituer un radical divalent. Parmi les diaminosilanes on citera:

. le bis diméthylamino diméthylsilane,
. le bis diméthylamino diéthylsilane,
. le bis dibutylamino diméthylsilane,
. le bis diméthylamino méthylphénylsilane.

Le composé à groupement aminosilylé sera une silylurée lorsque n sera égal a 1 et lorsque R' représentera le groupement carbonamide

```
-C-NRR'''
 |
 O
```

où R''' représente un radical R ou $SiR_3$, les radicaux R et R" ayant l'une des significations préalablement données, 2 radicaux R pouvant constituer ensemble un radical divalent. Parmi les silylurées on citera:

. la N, méthyl N trimétnylsilyl, N' méthyl N' butyl urée,
. le N triméthylsilyl N métnyl, N', N' diméthyl urée,

. la N triméthylsilyl N éthyl N', N' diméthyl urée,
. la N triméthylsilyl N butyl, N' butyl N' triméthylsilyl urée.

Le composé à groupement aminosilylé sera un silazane lorsque n sera égal à 1 et lorsque R' representera un groupement $SiR_3$.

Les silazanes peuvent être symétriques ou dissymétriques; on emploie préférentiellement les disilazanes symétriques, les deux groupements $SiR_3$ étant identiques.

Parmi les disilazanes utilisables on citera:

. l'hexaméthyldisilazane,
. l'heptaméthyldisilazane,
. le diéthyl-1,3 tétramétnyl-1,1,3,3 disilazane,
. le divinyl-1,3 tétraméthyl-1,1,3,3 disilazane,
. l'hexaéthyldisilazane,
. le diphényl-1,3 tétraméthyl-1,1,3,3 disilazane.

Enfin, on mentionnera tout particulièrement parmi les disilazanes l'hexaméthyldisilazane, l'heptaméthyldisilazane qui se révèlent être des catalyseurs tout particulièrement avantageux.

Le procédé de la présente invention peut être appliqué à la cyclotrimérisation en polyisocyanate polyisocyanurate de tout polyisocyanate simple ou adduct, de nature aliphatique, cycloaliphatique ou aromatique et ceci à condition de bien choisir, en tant que catalyseur, le composé à groupement aminosilylé apte à cette réaction.

C'est ainsi que la cyclotrimérisation catalytique de polyisocyanates simples ou adducts, et dont les groupements isocyanates ne sont pas directement reliés à un noyau aromatique, peut être aisément effectuée en mettant en oeuvre, en tant que catalyseur, un aminosilane, un diaminosilane, une silylurée ou un silazane, tels que précédemment définis.

Dans ce contexte, parmi les diisocyanates aliphatiques ou cycloaliphatiques, on citera:

. le tétraméthylène diisocyanate,
. le pentaméthylène diisocyanate,
. l'hexaméthylène diisocyanate,
. le diisocyanato-1,2 cyclohexane,
. le diisocyanato-1,4 cyclohexane,
. le bis(isocyante-méthyl)-1,2 cyclobutane,
. le bis(isocyanato-4 cyclohexyl)-méthane,
. le triméthyl-3,3,5 isocyanatométhyl-5 isocyanato-1 cyclohexane.

Parmi ceux-ci on mentionnera tout particulièrement l'hexaméthylène diisocyanate.

Enfin, on peut citer, parmi les polyisocyanates adducts ou prépolymères utilisables à titre de polyisocyanates de nature aliphatique, les polyisocyanates modifiés qui sont obtenus en faisant réagir un excès de polyisocyanate aliphatique ou cycloaliphatique sur un composé comportant au moins deux groupements réactifs vise-vis des groupements isocyanates, tel qu'une diamine, un diacide. Les polyisocyanates modifiés qui peuvent être mélangés avec des polyisocyanates simples peuvent comporter des groupements urée, biuret, ester, siloxane.

Le procédé de la présente invention peut également s'appliquer à l'interruption de la cyclotrimérisation en polyisocyanate polyisocyanurate de tout polyisocyanate simple ou adduct de nature aromatique, c'est-à-dire ceux dans lesquels le groupement NCO est directement lié à un groupement aromatique. Pour y parvenir, on utilisera, en tant que catalyseurs à groupements aminosilylés, les aminosilanes, les diaminosilanes ou les silylurées telles que précédémment définies.

Parmi les diisocyanates aromatiques utilisables, on citera:

. le diisocyanato-1,4 benzène,
. les diisocyanato-toluène(-2,4 et -2,6 ou bien leurs mélanges),
. le diisocyanato-4,4' diphénylméthane,
. le diisocyanato-4,4' diphényléther,
. les polyméthylène, polyphénylène polyisocyanates.

On peut également utiliser à titre de polyisocyanate aromatique tout polyisocyanate adduct résultant de la polycondensation d'un excès de polyisocyanate sur un composé polyfonctionnel tel qu'une diamine, un diacide. Les polyisocyanates modifiés qui peuvent être mélangés avec des polyisocyanates simples peuvent comporter des groupements urée, biuret, ester, siloxane.

La quantité d'agent catalytique introduite dans l'isocyanate peut être variable, celle-ci exprimée

pondéralement par rapport à l'isocyanate engagé étant habituellement comprise entre 0,1 et 10 % et de préférence entre 0,5 et 5 %; on peut éventuellement introduire de petites quantités complémentaires de catalyseur durant la réaction.

Le procédé de cyclotrimérisation en polyisocyanate polyisocyanurate peut être effectué par simple chauffage des réactifs, à une température, en général, comprise entre 50° C et 180° C, de préférence entre 80° C et 130° C et habituellement autour de 100° C.

Il est également possible, le cas échéant, d'effectuer la réaction de cyclotrimérisation en milieu solvant, ce dernier pouvant être un solvant peu polaire comme par exemple un hydrocarbure aliphatique ou aromatique, ou un ester ou un éther. On peut alors introduire le catalyseur dans le solvant et introduire cette solution dans l'isocyanate. On peut également introduire évidemment la solution catalytique dans l'isocyanate. Avantageusement, le procédé est effectué sans solvant.

Lorsque la teneur en isocyanurate atteint la valeur désirée, ajoute la quantité d'eau adéquate éventuellement après avoir amené la température du mélange de trimérisation à la valeur choisie.

On peut alors éventuellement éliminer le polyisocyanate monomère excédentaire par tout moyen connu et parvenir à un polyisocyanate polyisocyanurate renfermant une teneur excessivement réduite en isocyanate monomère, ainsi qu'une faible teneur en isocyanate dimère.

Les polyisocyanates polyisocyanurates tels que ceux issus d'hexaméthylène diisocyanate sont des composés bien connus et particulièrement intéressants comme constituants de base pour vernis et peinture.

Les exemples qui suivent illustrent l'invention.

**Exemple 1**

Dans un ballon de 3 l, équipé d'un agitateur et d'un thermomètre, on charge 1600 g de diisocyanato-1,6 hexane, soit 9,5 moles. On chauffe au bain d'huile à 100° et ajoute 16 g (0,1 mole) d'hexaméthyldisilazane; puis on porte à 120° C et on maintient cette température pendant 2 h 30 mn. Au bout de ce temps, on refroidit vers 50° C et on dose la teneur en groupes NCO, qui est alors de 0,973 groupe/100 g.

On ajoute alors, sous bonne agitation 0,9 g d'eau (0,05 mole) et on maintient ainsi pendant 10 à 15 minutes.

Une partie du mélange final est prélevée et maintenue 20 heures à 100° C: on constate qu'au bout de ce temps la teneur en NCO n'a pas varié, cequi montre que la réaction de trimérisation est bien arrêtée.

La masse réactionnelle restante est évaporée sous vide au moyen d'un évaporateur couche mince à film agité, jusqu'à obtention d'une teneur résiduelle en diisocyanate libre inférieure ou égale à 0,1 %. A près un mois de conservation à 60°, cette teneur n'a pratiquement pas variée (inférieure à 0,2 %).

**Exemple 2**

On réalise la même réaction que pour l'exemple 1; en fin de refroidissement à 50°, on divise la masse réactionnelle en deux parties égales:

a) à la première moitié, on ajoute 0,05 mole d'eau (soit 1 mole par mole de catalyseur) et on agite pendant 15 minutes.

b) à la deuxième moitié, on ajoute 0,0125 mole d'eau (soit 0,25 mole par mole de catalyseur) et on agite pendant 15 minutes.

Une partie de chacun de ces mélanges obtenus est prélevée et maintenue à 100° pendant 20 heures: la teneur en NCO reste constante dans les deux cas.

Le restant de ces deux mélanges réactionnels est évaporé sous vide avec un évaporateur à film agité, jusqu'à obtention d'une teneur résiduelle en diisocyanate libre inférieure ou au plus égale à 0,1 % en poids.

Après 1 mois de conservation à 60°, cette teneur n'a pratiquement pas varié pour le produit a) et a dépassé un peu 0,2 % pour le produit b).

**Exemple 3**

Dans un ballon de 3 litres, on charge 1600 g de diisocyanato-1,6 hexane (9,5 moles). On chauffe au bain d'huile à 100° et ajoute 14,5 g (0,1 mole) de N,N'-diéthylaminotriméthylsilane; on maintient 3 heures à 100° et refroidit ensuite vers 50°. La teneur en NCO est alors de 0,951 groupement pour 100 g.

Sous bonne agitation, on ajoute alors 0,9 g d'eau (0,05 mole) et on maintient ainsi pendant 20 minutes.

Comme dans l'exemple 1, on constate la stabilité de la teneur en NCO après 20 heures à 100° (teneur inchangée).

De même, le mélange réactionnel est évaporé et, après un mois de conservation à 60°, la teneur en diisocyanate libre n'a pratiquement pas varié.

**Revendications**

1. Procédé de préparation de polyisocyanates polyisocyanurates par cyclotrimérisation catalytique de polyisocyanates, en utilisant à titre de catalyseurs des composés à groupements aminosilylés puis, lorsque la teneur désirée en groupement isocyanurate est atteinte, destruction du catalyseur par addition d'un composé désactivateur caractérisé en ce que l'on utilise l'eau comme désactivateur.
2. Procédé selon la revendication 1, caractérisé en ce que la quantité d'eau est comprise entre 0,25 et 1 mole par groupe aminosilyle du catalyseur.
3. Procédé selon l'une quelconque des revendications 1 à 2, caractérisé en ce que la température d'addition de l'eau est comprise entre 0 et 100° C.

**Patentansprüche**

1. Verfahren zur Herstellung von Polyisocyanaten-Polyisocyanuraten durch katalytische Cyclotrimerisation von Polyisocyanaten unter Verwendung von Verbindungen mit Aminosilylgruppen als Katalysatoren, dann, wenn der gewünschte Gehalt an Isocyanuratgruppen erreicht ist, Zerstörung des Katalysators durch Zugabe einer entaktivierenden Verbindung, dadurch gekennzeichnet, daß man Wasser als Desaktivator verwendet.
2. Verfahren gemäß Anspruch 1, dadurch gekennzeichnet, daß die Wassermenge zwischen 0,25 und 1 Mol pro Aminosilylgruppe des Katalysators beträgt.
3. Verfahren gemäß einem der Ansprüche 1 bis 2, dadurch gekennzeichnet, daß die Zugabetemperatur des Wassers zwischen 0 und 100° C liegt.

**Claims**

1. Process for the preparation of polyisocyanurates polyisocyanates by catalytic cyclotrimerization of polyisocyanates, with the use of compounds containing aminosilyl groups as catalysts, followed, when the required isocyanurate group content has been obtained, by destruction of the catalyst by the addition of a deactivating compound, characterized in that water is used as the deactivator.
2. Process according to claim 1, characterized in that the quantity of water is between 0.25 and 1 mole per aminosilyl group in the catalyst.
3. Process according to either of claims 1 to 2, characterized in that the temperature of addition of water is between 0 and 100° C.